# EUROPEAN PATENT APPLICATION

(11) **EP 1 530 950 A1**
(43) Date of publication of application: **18.05.2005**
(21) Application number: 03104160.1
(22) Date of filing: 12.11.2003
(51) Int. Cl.: A61B 10/00

(54) **Sampling device and method of producing thereof**

(71) Applicant: Färgklämman AB, 141 75 Huddinge (SE)
(72) Inventor: Stoltz, Klas, 133 36, Saltsjöbaden (SE)
(74) Representative: Hagström, Lena

(57) **Abstract**

The present invention relates to a sampling device (2) for obtaining samples of substances, which device (2) allows a sample of the substance to enter the device (2) through inlet openings (4) into an inner chamber (8) of the device (2). The inlet openings (4) are provided with sealing means (3) that is disposed to preserve a vacuum or substantial underpressure in the inner chamber (8) when the inlet openings (4) are sealed. The sealing means (3) is adapted to be in a sealing position at a first temperature range in which flow (6) of substance through the inlet openings (4) is prevented and is adapted to be in an opening position at a second temperature range in which flow (6) of substance is permitted through said inlet openings (4) and into the inner chamber (8).

## Description

### Field of the invention

The present invention relates to a sampling device for obtaining samples of substances in places where sampling is particularly inaccessible, for instance the digestive system of humans or animals, the sample obtained being a sample of internal body substance. The sampling device allows a sample of the substance to enter the device through a number of inlet openings which are sealed at a first temperature range and open at second temperature range. The invention also relates to a method of producing the sampling device and use of said sampling device for obtaining samples of internal body substances.

### Background of the invention

Obtaining samples of substances in places which are particularly inaccessible is a problem in many situations. For instance the sampling of internal body substances, gases or solid particles in the digestive system or the gastrointestinal tract in the human or animal body. These substances provide essential medical information for diagnosing and treatment. Examination of a sample of the gastric fluid of a patient provides important information of pH, acid contents, abdominal enzyme activity as well as information for diagnosing gastric ulcer and gastritis, cancer and tumour diseases, etc. A gastroscopic examination gives the physician who is treating a patient important information and plays a great role for a diagnosis. These intubation examinations are thus used extensively. A gastroscopic examination, in which a tube, having a diameter of a little finger, is inserted into the patient's mouth or nose, through the oesophagus and to the gastrointestinal system, is difficult to perform and demands the assistance of a physician. For the patient, the intubation of the digestive tract using these methods is a very unpleasant intervention, both physically and psychologically, especially during the insertion of the tube and also when it is pulled out. The intubation demands that the patient is given a local anaesthetic and in some instances even tranquilliser or a general anaesthetic to overcome the stress to which the patient is subjected. The intubation examination methods described above are disadvantageous since they are very time-consuming for a qualified physician and thus expensive and a very unpleasant intervention for the patient.

WO 02/102983 A1 describes a sampling device for obtaining samples of internal body substances, which allows a sample of the body substance to enter the device through an inlet opening which is opened at predetermined position of the digestive tract following contact with the body substance to be collected. This is accomplished through the sealing of the inlet opening with a plug made of a material which is chosen depending on the specific body fluid to be collected. Following contact with said fluid the plug is dissolved and fluid flows into the device by the force of a substantial underpressure in the device. When the device is filled, the inlet opening is automatically closed, sealing it from the inside of the device, such that the sample remains in the device. This is accomplished through an elastic blocking member , resiliently movable between a flow permitting and a flow preventing configuration.

The sampling device of WO 02/102983 A1 is advantageous in many ways. However, the method of producing the device requires the assembling of the different parts of the device in a vacuum chamber, which results in unnecessarily complicated production steps and thus high production costs.

### Summary of the invention

The object of the present invention is to provide a sampling device being just as reliable and easy to use as the sampling device of WO 02/102983 A1 but which is simpler and less expensive to produce.

The sampling device according to the present invention is not limited for use in the digestive system or the gastrointestinal tract in the human or animal body, but can be used for obtaining samples of substances in many otherwise inaccessible places.

These objects are achieved by a device according to the preambles of the independent claims and provided by the features according to the characterising portions of the independent claims.

Preferred embodiments are set forth in the dependent claims.

Moreover, if the device of the present invention would get caught in the body of the patient, it contains, according to a preferred embodiment, a minor part of an X-ray opaque material which ensures that the position of the sampling device is detectable during an X-ray examination.

In accordance with the present invention, the sampling device is provided with sealing means that, when in a first temperature range, is in a sealing position of a number of inlet openings of the sampling device. When subjected to a second temperature range the sealing means is moved to an opening position of the inlet openings. The sample of the substance to be taken flows into an inner chamber of the device through the inlet openings by the force caused by a vacuum or substantial underpressure in the inner chamber when the sealing means is in the opening position.

The sampling device according to the invention is simple and is made of a few parts. The parts can be assembled in practically any environment of choice, with only one step requiring vacuum.

### Short description of the drawings

The invention will be described in greater detail below with reference to the attached drawings, in which:
Figure 1 is a schematic sectional view of a sampling device of the invention, in a first closed and sealed state,
Figure 2 shows the sampling device of Figure 1 in a second, open state, with the substance flowing into the device,
Figure 3 shows the top part of the sampling device, partly from above.
Figure 4 shows a schematic sectional view of the sampling device according to a preferred embodiment of the present invention,
Figure 5 shows a schematic sectional view of the sampling device in an alternative embodiment of the present invention,
Figure 6 shows the membrane, from above,
Figure 7 shows the pushing means partly from above.
Figure 8 shows a schematic sectional view of the sampling device in a second preferred embodiment,
Figure 9 shows a schematic sectional view of the sampling device in a second alternative embodiment,
Figure 10 shows the sampling device in a third alternative embodiment with the device having inlet openings at each side.

### Detailed description of embodiments of the invention

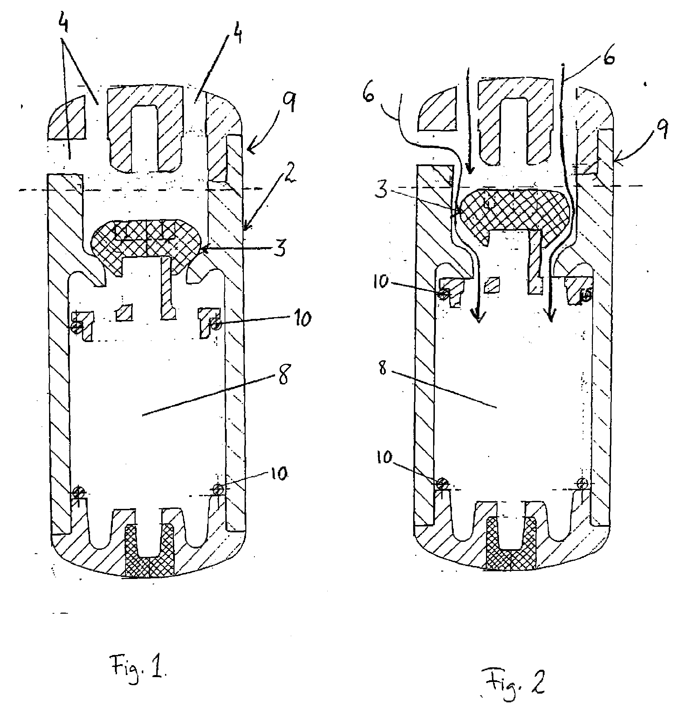

As seen from Figure 1 the sampling device 2 is preferably elongated with rounded end portions and preferably has a circular or oval-shaped cross-section. The device 2 further comprises sealing means 3 which is in a sealing position of inlet openings 4 of the device 2 when said device 2 is in the closed and sealed state and which is in an opening position of the inlet openings 4 when the device 2 is in the open state shown in Figure 2, in which open state a flow 6, indicated by arrows, of substance is permitted through the inlet openings 4 and into an inner chamber 8 of the device 2, defined by a device wall and the sealing means 3. As long as the device 2 is in the closed and sealed state, a vacuum or substantial underpressure prevails in the inner chamber 8 but when the device 2 is in the open state, a suction effect is generated by the pressure difference between the external environment of the device 2 and the inner chamber 8. This suction effect forces substance in the external environment of the device 2 to flow 6 into the inlet openings 4.

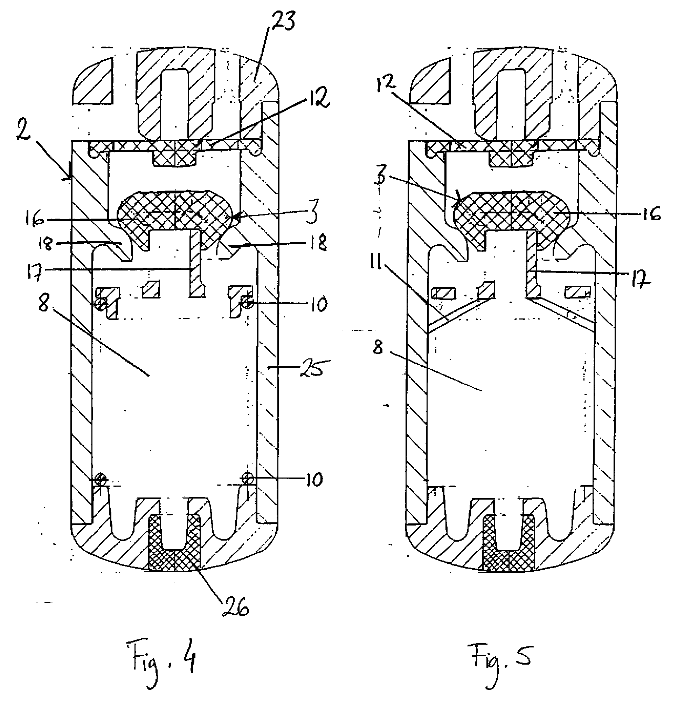

As seen from Figure 3, a number of inlet openings 4 are arranged on the top part 9, marked out by the dashed lines in Figure 1-2, as well as along the circumference of the mantle part of the top part 9. Preferably, the inlet openings are equally distributed on the top part 9. The number of inlet openings is preferably 1-10.

The movement of the sealing means 3 from the sealing position to the opening position of the inlet openings 4 is accomplished by a temperature activated element 10, 11 in Figure 1-2, 4, 8-10 and Figure 5, respectively. The temperature activated element 10, 11 is made of a material, which upon reaching a certain temperature range undergoes a predefined deformation.
Preferably, the deformed shape will be kept until the material returns to the original temperature range, i.e. the first temperature range, in which the material regains it original shape. Such a material is known as a memory element with a two way memory, and is for instance a metal, a bimetal, an alloy of for instance TiNi or a polymer. The deformation can be for instance an elongation, a shortening, a bending, a torsion etc. The temperature at which the material starts to deform, i.e. lower limit of the second temperature range, varies from one material to another but lies within the wide temperature range of -20°C to + 120°C.

The temperature activated element 10, 11 can also be made of a memory element material with a one way memory. In this case, the temperature activated element 10, 11 undergoes said deformation upon reaching the lower limit of the second temperature range but will not regain its original shape when returning to the first temperature range but stay in the deformed shape.

A preferred embodiment of the present invention is shown in Figure 4, in the closed an sealed state, i.e. when within the first temperature range. In this embodiment the temperature activated element 10 is in the shape of a helix-formed spring comprised in the inner chamber 8 of the device 2. When the device is subjected to the second temperature range, the temperature activated element 10 performs a movement, acting on the sealing means 3 such that it moves from the sealing position to the opening position. The movement is in this case the elongation of the temperature activated element 10 in the shape of a helix-formed spring.

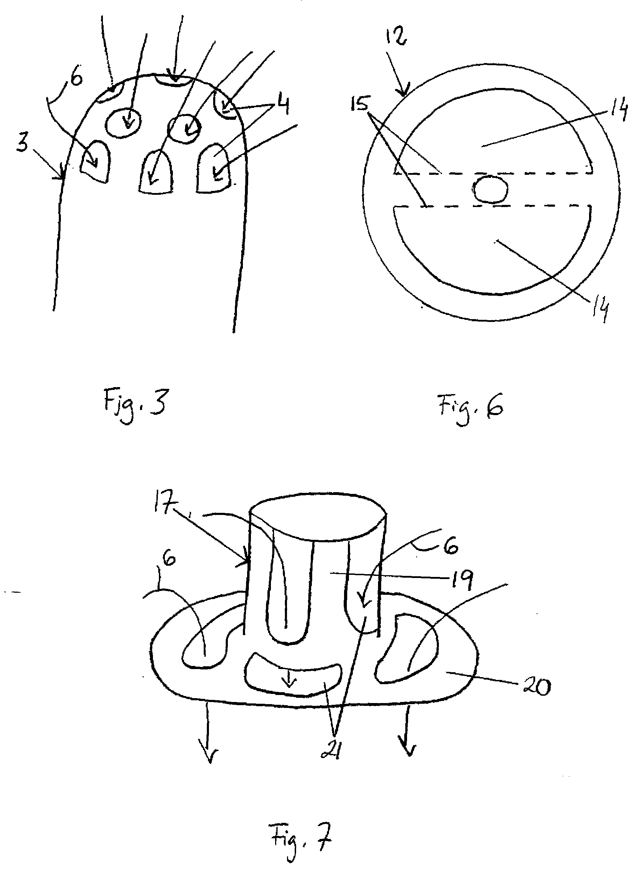

Figure 5 shows an alternative embodiment of the present invention in which the temperature activated element 11 is in the shape of one or many rods, for instance, three or four rods 11 are fastened in the same plane at the inner surface of the inner chamber 8 at equally distributed positions. Preferably, the rods 11 are attached by welding or brazing approximately in the radial direction at the inner periphery of a circular ring-shaped member (not shown) supported in a recess of the inner wall of the inner chamber 8. The movement in this case, i.e. when the device 2 is subjected to the second temperature range, is the upwards directed bending of the rods 11, moving the sealing means 3 from the sealing position to the opening position.

In the preferred embodiment the helix-formed spring 10 or in the alternative embodiment the rods 11 are made of a temperature activated material with a two way memory and regain the shape they had before being subjected to the second temperature range when once again subjected to the first temperature range, the movement acting on the sealing means 3 which causes it to move to the sealing position.

There are of course several other possible designs of the temperature activated element, regardless whether the temperature activated material as a one way memory or a two way memory, provided that the temperature activated element is able to perform the movement acting on the sealing means moving it from the sealing position to the opening position. Naturally, the structural design of the sealing means 3 and of the temperature activated element 10, 11 are adopted to each other.

In the preferred embodiment shown in Figure 4, a membrane 12 having a two-way valve function is positioned between the inlet openings 4 and the sealing means 3. The membrane 12, as seen in greater detail in Figure 6, is preferably a disc made of an elastic material, such as SiO₂, provided with one or more foldable, normally sealed, openings achieved by foldable parts 14. When the sealing means 3 moves to the opening position, the suction effect generated by the pressure difference between the external environment of the device 2 and the inner chamber 8, causes the foldable parts 14 to fold downwards along the dashed lines 15 admitting flow 6 of substance into the inner chamber 8 as long as there is a pressure difference between the external environment of the device 2 and the inner chamber 8. As the external and internal pressure of the device 2 equalise and the substance fills up the inner chamber 8, the foldable parts 14 gradually folds back to its original shape. As soon as the substance has filled up the inner chamber 8, the membrane secures that essentially all flow of substance out from the inner chamber 8 is prevented.

Moreover, in the preferred embodiment shown in Figure 4, the sealing means 3 is made of two parts, i.e. a sealing part 16 and a pushing part 17. The sealing part 16 is preferably somewhat elastic to ensure a tight fit against protrusions 18 on the inner side of the device wall and the pushing part 17, which is the part that the temperature activated element 10, 11 acts on, are more firm. Naturally, the sealing means 3 can be made in one piece that includes the sealing part 16 and the pushing part 17.

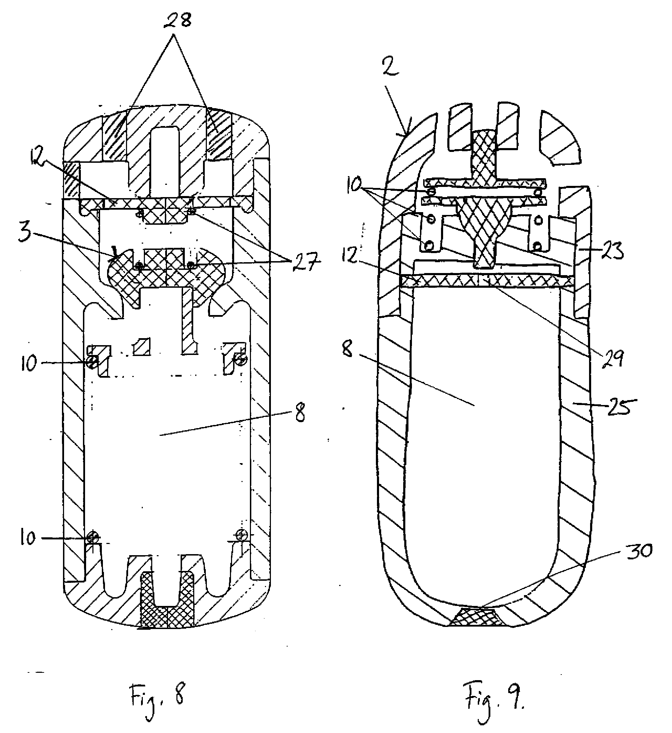

Figure 7 shows the pushing part 17 in greater detail. The pushing part 17 preferably has the shape of a "top hat", i.e. a hollow cylindrical part 19 with a wider bottom or "brim" 20. The pushing part 17 has openings 21 equally distributed around the brim 20 as well as around the mantle of the cylindrical part 19, through which openings 21 substance is flowing into the inner chamber 8 of the device 2 when the sealing means 3 is in the opening position and as long as a pressure difference prevails between the external environment of the device 2 and the inner chamber 8.

Moreover, in the preferred embodiment shown in Figure 4 as well as in the second alternative embodiment of Figure 9, the device 2 is made of a cap member 23 and a body member 25 to facilitate assembling of the device which will be described below. However, these members 23, 25 can naturally be in one piece.

Also shown in the preferred embodiment of Figure 4, a recess 26 is made in a section of the device wall, preferably at the bottom end, which is filled with for instance a soft, easily penetratable material, such as silicon rubber. This recess 26 can be used when the substance in the inner chamber 8 shall be evacuated from the device 2. An evacuation needle then, directly or indirectly connected to an instrument for examination and analysis of the substance, penetrates the recess 26 of the chamber wall and empties the inner chamber 8. The recess 26 can also be made not all the way through the device wall but saving a thin partition 30 of said wall, as seen in Figure 9, which partition 30 is thin enough to be penetrated by conventionally used evacuation needles. The recess 26 seen in Figure 9, is optionally filled with the above mentioned soft, easily penetratable material.

In a second preferred embodiment shown in figure 8, pushing means 27, preferably in the shape of a helix-formed spring, is arranged between the membrane 12 and the sealing means 3. The pushing means 27 is in a relaxed state when the sealing means 3 is in the sealing position but when the sealing means 3 moves to the opening position the pushing means 27, is forced to a tensed state. Thus, when the sealing means 3 is about to return to the sealing position, i.e. when the substance has filled up the inner chamber 8 and/or when the device 2 is subjected to the first temperature range, the pushing means 27 spontaneously assists the movement of the sealing means 3 back to the sealing position. This is particularly preferred when the temperature activated element 10, 11 is made of a memory element material with a one way memory, since the temperature activated element 10, 11 spontaneously will not regain its original shape when once again being subjected to the first temperature range when being in the second temperature range. The pushing means 27 can of course have other possible designs other than in the shape of a helix-formed spring, as long as it is adopted to spontaneously assist the movement of the sealing means 3 to the sealing position. For instance in the shape of one or more resilient rods.

Moreover, also shown in the second preferred embodiment of Figure 8, plugs 28 are arranged in the openings 10. A plug 28 is made of at least one material which is chosen depending on the application of device and which will dissolve upon contact with the substance that is to flow 6 into the inner chamber 8 of the device 2. This will be described in greater detail below. Naturally, the plugs 28 are not restricted to be arranged in the embodiment of the device 2 shown in Figure 8, but can be arranged in all embodiments of the present invention.

A second alternative embodiment is shown in Figure 9, in which the temperature activated element 10 is positioned above the inner chamber 8 and the membrane 12 is positioned inside the inner chamber 8. In this embodiment the membrane 12 may have the shape of a disc that has a slit 29, preferably at the centre, which admits flow 6 of substance into the inner chamber 8 as long as there is a pressure difference between the external environment of the device 2 and the inner chamber 8. As soon as the external and internal pressure of the device 2 have been equalised and the substance has filled up the inner chamber 8, the membrane 12 secures that flow of substance out from the inner chamber 8 is prevented.
Naturally, in this embodiment the temperature activated element can be in the form of rods 11 as shown in Figure 5, and the membrane 12 can be positioned between the sealing means 3 and the inlet openings 4 as in the preferred embodiment of Figure 4. Likewise, the membrane 12 can have the design as shown in Figure 6.

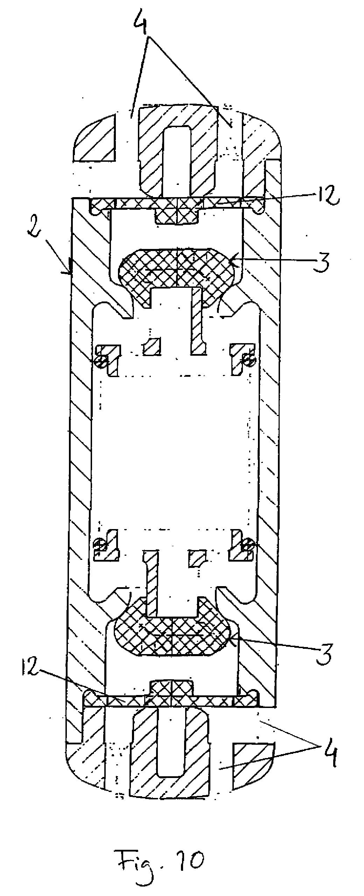

A third alternative embodiment of the device 2 is shown in Figure 10. In this embodiment the device has a second inlet openings 4, provided with second sealing means 3 and a second membrane 12. The sampling device 2 has an elongated cylindrical form with a first end and a second end, with said second inlet openings 4, said second sealing means 3 and said second membrane 12 provided in said second end.

In a fourth alternative embodiment of the invention, protrusions are disposed externally on the device 2 at the inlet openings 4, not shown in any figure, such that laterally directed inlet grooves are formed between the protrusions.

In a fifth alternative embodiment, not shown in any figure, pulling means is arranged in the upper part of the device 2 in connection with the sealing means 3. The pulling means consists of, for instance a spring, which is in the tensed state when the sealing means 3 is in the sealing position. The spring or the like is held in the tensed state by holding means, for instance one or more threads made of a material which is chosen depending on the application of device. This material will dissolve upon contact with the substance that is to flow 6 into the inner chamber 8 of the device 2, which results in the pulling means shifting to its relaxed state, pulling the sealing means 3 to the opening position. The material of the thread is in the preferred use, i.e. collecting a sample of internal body substance from the digestive system of humans or animals, for example, gelatine, molten sugar, salt, glue, organic edible materials or any other suitable material. In this fifth alternative embodiment, the device 2 is preferably provided with the membrane 12 inside the inner chamber 8. The membrane 12 having the design as shown in Figure 6 or as explained in connection with Figure 9.

When collecting a sample of a substance, the sampling device 2 is put at the particular place from which environment a sample is to be taken. This place must secure that the temperature activated element 10, 11 reaches the said second temperature range, at which the element performs the above mentioned deformation, such that the sealing means 3 moves to the opening position of the inlet openings 4. As soon as the substance has filled up the inner chamber 8, the device 2 can be removed from the place where the sample was taken. If the temperature activated element 10, 11 is made of a temperature activated material with a two way memory, the temperature activated element 10, 11 regains its original shape when again subjected to the first temperature range and the sealing means 3 moves to the sealing position of the inlet openings 4. If the temperature activated element 10, 11 is made of a temperature activated material with a one way memory, the membrane 12 still secures flow of substance out from the inner chamber 8 of the device 2.

In a preferred use of the sampling device 2 according to the present invention, the substance to be taken is a sample of internal body substance from the digestive system of humans or animals. The sampling device 2 preferably having the shape of a swalloable capsule, such as a conventional pharmaceutical capsule, and thus suitable for ingestion. When an examination of a body fluid in the digestive system is required, the patient swallows the sampling device 2, which is in the closed and sealed state of Figure 1, without any stress or pain. The device 2 passes the oesophagus and enters into the stomach. During the passage of the device 2 from the mouth to the stomach the patient feels no more discomfort than he feels when he swallows an ordinary pharmaceutical capsule, i.e. a discomfort which is principally non-existing.

The sampling device 2 is according to the present invention provided with features preventing it from getting stuck, for instance in the intestines, by the suction forces generated by the internal underpressure. This is achieved by inlet openings 4 distributed along the circumference of the mantle part of the top part 9 of the device 2 that secure that flow of substance always is permitted and accordingly the device 2 will not get stuck by the forces of vacuum inside the device 2. Moreover, by having several inlet openings 4 it is secured that substance may enter the device 2 even though one of the inlet openings 4 is being blocked by for instance a large particle of some sort.

Also the third alternative embodiment seen in Figure 10 with the device 2 having inlet openings 4 at the first and at the second end of the device, secure the device 2 from being adhered to, for instance the walls of the digestive system in a similar way.

Even the fourth alternative embodiment with protrusions disposed externally on the device 2 at the inlet openings 4, prevent the device 2 from getting stuck since the protrusions always secure a free flow of substance through the inlet grooves into the device 2.

The size of the device 2 may vary depending on the volume of body fluid required for the examination and analysis. A suitable size of a device 2 which is easy to swallow has, for example, a length of about 25 mm and a width of about 10 mm. Thus, a typical volume that is to be collected is approximately 0,4-1 ml.

In the preferred use the temperature activated element 10, 11 needs to be of such a material, that the temperature of the human or animal body, that is typically 35-40°C, is within the second temperature range of said temperature activated material, so that the sealing means 3 moves from the sealing position to the opening position in the gastrointestinal tract of the body. The device 2 then is filled up with body substance, in a way previously described, is fed through the gastrointestinal tract and is recovered from the fecies of the patient. Once outside of the body, the temperature activated element 10, 11 is subjected to the first temperature range and, if the temperature activated element 10, 11 is made of a temperature activated material with a two way memory, regains its original shape and the sealing means 3 moves to the sealing position. If the temperature activated element 10, 11 is made of a temperature activated material with a one way memory the membrane 12 still secures flow of substance out from the inner chamber 8 of the device 2 and if the device 2 is provided with the pushing means 27 as in the second preferred embodiment, said pushing means 27 will assist the movement of the sealing means 3 back to the sealing position.

As mentioned, the inlet openings 4 can in an alternative embodiment further be sealed by plugs 28 (Figure 8). The plug 28 in the device 2 is made of a material which is chosen depending on the application of the device 2, in the preferred use i.e. depending on the specific body fluid to be collected, in which body fluid the plug 28 is dissolved after a short period of time. Thus, the material of the plug 28 is adapted to the specific fluid or substance in the external environment of the device 2 where the sample is to be collected. The material of the plug 28 is in the preferred use, for example, gelatine, molten sugar, salt, glue, organic edible materials or any other suitable material. The plug 28 can also be made of two or more layers of different materials, which dissolve gradually upon contact with different substances in the external environment of the device 2, for instance during its passage through the digestive system. The innermost layer dissolving upon contact with substance to be collected by the device 2. Alternatively, instead of having plugs 28, the entire device 2 can be coated with the material(s) described above. Either way, the sealing means 3 moves from the sealing position to the opening position when reaching the second temperature range as previously described.

The sampling device 2 may initially be provided with a substance in the inner chamber 8, which substance is used in the analyzing of the substance of which a sample is taken.

The sampling device 2 according to the invention is in the preferred embodiment made of a few parts, i.e. the cap member 23, the body member 25, the sealing means 3, the membrane 12, the temperature activated element 10, 11 and optionally the plugs 28. All these parts, apart from the plugs 28, are initially fully or partly assembled in practically any environment of choice. The cap member 23 and the body member 25 device are permanently joined together, preferably by ultrasonic welding and are preferably made of injection-moulded thermoplastic, such as Macrolon® D for example, which is a strong, acid resistant and transparent material and thus suitable for the preferred use. The mounting of the sealing means 3, the membrane 12 and the temperature activated element 10, 11 is carried out with conventional methods.

The assembly-step that finally seals off the inner chamber 8 of the device 2 from the external environment is however carried out in an vacuum-oven or the like, i.e. a device that is settable at predetermined temperatures and which at the same time is capable to create and maintain a vacuum. This is in order to achieve a vacuum or substantial underpressure in the inner chamber 8, such that sufficient suction forces are generated to suck substance into and fill the device 2 when in use.

The assembled device 2, comprising the above mentioned components, is arranged in a vacuum-oven, either previously set at a temperature within the second temperature range or the setting of said temperature is performed after the arranging of the device 2. Also, the device 2 can already have a temperature that is within the second temperature range before being arranged in the oven. Either way the temperature of the vacuum-oven being in the second temperature range results in the sealing means 3 being in the opening position of the inlet openings 4. In connection to the temperature setting, a vacuum is established in the oven. This can be done prior to, or after the temperature setting, as long as the sampling device 2 is arranged in the vacuum-oven. Subsequently, the temperature is set to be within the first temperature range of the temperature activated material while maintaining the vacuum. If the temperature activated element 10, 11 is made of temperature activated material with a two way memory, this will result in the sealing means 3 moving to the sealing position of the inlet openings 4, sealing off the inner chamber 8. If the temperature activated element 10, 11 is made of temperature activated material with a one way memory, then the device 2 preferably is provided with the pushing means 27 that will assist the movement of the sealing means 3 back to the sealing position, as previously described in connection to Figure 8. Either way, the device 2 can be removed from the vacuum-oven when the sealing means 3 is in the sealing position and the inner chamber 8 of the device 2 has a vacuum or substantial underpressure.

Said vacuum or substantial underpressure inside the inner chamber 8 may not have a magnitude greater than that the temperature activated element 10, 11 can overcome the forces that keep the sealing means 3 in the sealing position and move this to the opening position when said element 10, 11 adopts a temperature within the second temperature range. A typical magnitude of said vacuum being ca 0,1 bar.

However, the area over which the forces generated by the vacuum act is small in comparison with the total cross section of the device 2. Thus the magnitude of the vacuum in the inner chamber 8 of the device 2 may very well be greater than that. Moreover, the greater the difference between the temperature within the first temperature range at which the sampling device initially is at, i.e. the temperature that the temperature activated element 10, 11 initially has, and the origin of the second temperature range, the greater the forces that will act on the sealing means 3 generated by the temperature activated element 10, 11 when subjected to the second temperature range. Therefore, for the preferred use, it might be necessary to store the sampling device in for instance a refrigerator, i.e. typically 4°C, and keep the device at this temperature just prior to the swallowing of said device in order to generate a temperature difference of approximately 30°C.

In a final step the plugs 28 can optionally be applied with conventional methods without the need of a vacuum-chamber.

The device 2 may further be provided with an additional outer coating. In the preferred use this might for instance be a covering film of gelatine or any other suitable material, which is dissolved in the digestive system. The film may be applied in order to minimise the resistance when the device 2 is swallowed by the patient.

It will be understood that the invention is not restricted to the above-described exemplifying embodiments thereof and that several conceivable modifications of the invention are possible within the scope of the following claims.

For instance the temperature activated element 10, 11 can have practically any design as long as the deformation of said element 10, 11 upon reaching the second temperature secures the movement of the sealing means 3 to the opening position.

Also the design of the sealing means 3 and the membrane 12 may vary from the description and the drawings of this present application as long as they perform the functions of the present invention which is defined by the appended claims.

## Claims

1. A sampling device (2) for obtaining samples of substances, which device (2) allows a sample of the substance to enter the device (2) through at least one inlet opening (4) into an inner chamber (8) defined by a device wall, said inlet openings (4) is provided with at least one sealing means (3) that is disposed to preserve a vacuum or substantial underpressure in the inner chamber (8) when the inlet opening (4) is sealed **characterised in that** said sealing means (3) is adapted to be in a sealing position at a first temperature range in which flow (6) of substance through the inlet opening (4) is prevented and is adapted to be in an opening position at a second temperature range in which flow (6) of substance is permitted through said inlet opening (4) and into the inner chamber (8), wherein the device (2) comprises at least one temperature activated element (10, 11) made of at least one temperature activated material, which is adapted to move the sealing means (8) from the sealing position to the opening position when subjected to the second temperature range.

2. A sampling device according to claim 1, **characterised in that** the temperature activated element (10, 11) is adapted to move the sealing means (3) from the opening position to the sealing position when subjected to the first temperature range.

3. A sampling device according to any of claims 1-2, **characterised in that** a degree of 35°C is within the second temperature range and that a degree of 25°C is within the first temperature range.

4. A sampling device according to any of claims 1-3, **characterised in that** said temperature active material is a material with a two way memory.

5. A sampling device according to any of claims 1-4, **characterised in that** said temperature active material is a memory metal and/or memory alloy.

6. A sampling device according to any of claims 1-5, **characterised in that** the temperature activated element (10) is a helix-shaped spring.

7. A sampling device according to any of claims 1-6, **characterised in that** there are several inlet openings arranged along the circumference of the mantle part of the top part (9) of the device (2).

8. A sampling device according to claims 1-7, **characterised in that** the sealing means (3) comprises a sealing part (16) and a pushing part (17).

9. A sampling device according to claims 1-8, **characterised in that** said device (2) is provided with at least one membrane (12), arranged such that it allows a flow (6) of substance into the inner chamber (8).

10. A sampling device according claim 9, **characterised in that** the membrane (12) is made of an elastic material.

11. A sampling device according to claims 1-10, **characterised in that** said device (2) is provided with pushing means (27) which is adapted to assist the movement of the sealing means (3) from the opening position to the sealing position.

12. A sampling device according to claim 11, **characterised in that** said pushing means (27) is in a relaxed state when the sealing means (3) is in the sealing position and in a tensed state when the sealing means (3) is in the opening position.

13. A sampling device according to claims 1-12, **characterised in that** the device (2) initially comprises at least one substance, which is used in the analyzing of the substance of which a sample is taken.

14. A method for producing a sampling device according to any of the preceding claims **characterised in that** said method comprises the steps (a) -(d) in arbitrary order, provided that step (a) is previous to step (c) and step (d) is the last step, said steps (a)-(d) being:
(a) arranging the sampling device (2) in a vacuum chamber,
(b) changing the temperature of the vacuum chamber to said second temperature range in which the sealing means (3) is in the opening position,
(c) lowering the pressure to a preset pressure level,
(d) changing the temperature to said first temperature range in which the sealing means (3) is in the sealing position,

15. A method according to claim 14 **characterised in that** said preset pressure level is 0 - 0,8 bar, preferably 0,1 bar.

16. Use of the sampling device according to any of the claims 1-13 **characterised in that** said sampling device (2) is used for sampling of body substances from the digestive tract of a patient.
